# EUROPEAN PATENT SPECIFICATION

(11) **EP 1 767 164 B1**
(45) Date of publication and mention of the grant of the patent: **09.01.2013**
(21) Application number: 05020666.3
(22) Date of filing: 22.09.2005
(51) Int. Cl.: A61B 18/14

(54) **Electrode assembly for tissue fusion**
Elektrodenanordnung zur Gewebeverschmelzung
Assemblage d'électrodes pour la fusion de tissus

(43) Date of publication of application: 28.03.2007
(73) Proprietor: Covidien AG, 8212 Neuhausen am Rheinfall (CH)
(72) Inventor: Hammill, Curt, Erie, Colorado 80516 (US); Odom, Darren, Golden, Colorado 80403 (US); Schechter, David A., Longmont, Colorado (US); Tetzlaff, Philip M., Superior, Colorado (US); Roy, Jeffrey M., Boulder, Colorado (US)
(74) Representative: HOFFMANN EITLE

(56) References cited:
- EP-A- 0 875 209
- EP-A- 0 986 990
- WO-A-2004/082495
- US-A1- 2005 033 278

## Description

### BACKGROUND

The present disclosure relates to forceps used for open and/or endoscopic surgical procedures. More particularly, the present disclosure relates to a forceps which applies a unique combination of mechanical clamping pressure and electrosurgical current to micro-seal soft tissue to promote tissue healing. The features of the pre-characterising portion of claim 1 are disclosed in WO-A-2004/082495.

### Technical Field

A hemostat or forceps is a simple plier-like tool which uses mechanical action between its jaws to constrict vessels and is commonly used in open surgical procedures to grasp, dissect and/or clamp tissue. Electrosurgical forceps utilize both mechanical clamping action and electrical energy to effect hemostasis by heating the tissue and blood vessels to coagulate, cauterize and/or seal tissue. The electrode of each opposing jaw member is charged to a different electric potential such that when the jaw members grasp tissue, electrical energy can be selectively transferred through the tissue. A surgeon can either cauterize, coagulate/desiccate and/or simply reduce or slow bleeding, by controlling the intensity, frequency and duration of the electrosurgical energy applied between the electrodes and through the tissue.

For the purposes herein, the term "cauterization" is defined as the use of heat to destroy tissue (also called "diathermy" or "electrodiathermy"). The term "coagulation" is defined as a process of desiccating tissue wherein the tissue cells are ruptured and dried. "Vessel sealing" is defined as the process of liquefying the collagen, elastin and ground substances in the tissue so that it reforms into a fused mass with significantly-reduced demarcation between the opposing tissue structures (opposing walls of the lumen). Coagulation of small vessels is usually sufficient to permanently close them. Larger vessels or tissue need to be sealed to assure permanent closure.

Commonly-owned U.S. Application Serial Nos. PCT Application Serial No. PCT/US01/11340 filed on April 6, 2001 by Dycus, et al. entitled "VESSEL SEALER AND DIVIDER", U.S. Application Serial No. 10/116,824 filed on April 5, 2002 by Tetzlaff et al. entitled "VESSEL SEALING INSTRUMENT" and PCT Application Serial No. PCT/US01/11420 filed on April 6, 2001 by Tetzlaff et al. entitled "VESSEL SEALING INSTRUMENT" teach that to effectively seal tissue or vessels, especially large vessels, two predominant mechanical parameters must be accurately controlled: 1) the pressure applied to the vessel; and 2) the gap distance between the conductive tissue contacting surfaces (electrodes). As can be appreciated, both of these parameters are affected by the thickness of the vessel or tissue being sealed. Accurate application of pressure is important for several reasons: to oppose the walls of the vessel; to reduce the tissue impedance to a low enough value that allows enough electrosurgical energy through the tissue; to overcome the forces of expansion during tissue heating; and to contribute to the end tissue thickness which is an indication of a good seal. It has been determined that a typical sealed vessel wall is optimum between 0.001 inches and 0.006 inches. Below this range, the seal may shred or tear and above this range the lumens may not be properly or effectively sealed.

With respect to smaller vessels, the pressure applied become less relevant and the gap distance between the electrically conductive surfaces becomes more significant for effective sealing. In other words, the chances of the two electrically conductive surfaces touching during activation increases as the tissue thickness and the vessels become smaller.

As can be appreciated, when cauterizing, coagulating or sealing vessels, the tissue disposed between the two opposing jaw members is essentially destroyed (e.g., heated, ruptured and/or dried with cauterization and coagulation and fused into a single mass with vessel sealing). Other known electrosurgical instruments include blade members or shearing members which simply cut tissue in a mechanical and/or electromechanical manner and, as such, also destroy tissue viability.

When trying to electrosurgically treat large, soft tissues (e.g., lung, intestine, lymph ducts, etc.) to promote healing, the above-identified surgical treatments are generally impractical due to the fact that in each instance the tissue or a significant portion thereof is essentially destroyed to create the desired surgical effect, cauterization, coagulation and/or sealing. As a result thereof, the tissue is no longer viable across the treatment site, i.e., there remains no feasible path across the tissue for vascularization.

Thus, a need exists to develop an electrosurgical forceps which effectively treats tissue while maintaining tissue viability across the treatment area to promote tissue healing.

A need exists also to enhance sealing strength in tissue fusion by increasing resistance to fluid flow or increased pressure at the fusion site so as to minimize entry of fluid into the perimeter of the fused site during burst strength testing. The entry of fluid often results in seal failure due to propagation of the fluid to the center of the tissue seal.

### SUMMARY

It is an object of the present disclosure to provide a bipolar electrosurgical forceps having jaw members which are configured with electrode surfaces with a plurality of flow paths so as to increase resistance to fluid flow through the tissue seal zone, or increasing pressure states at the fusion site, thereby increasing tissue seal integrity.

The present disclosure relates to a bipolar electrosurgical forceps which includes first and second opposing jaw members having respective tissue engaging surfaces associated therewith. The first and second jaw members are adapted for relative movement between an open position to receive tissue and a closed position engaging tissue between the tissue engaging surfaces to effect a tissue seal upon activation of the forceps. The first and second jaw members each include an electrode having a plurality of tissue engaging surfaces which define at least one channel therebetween. The plurality of tissue engaging surfaces of the first jaw member are substantially aligned with the plurality of tissue engaging surfaces of the second jaw member so as to impede fluid flow therebetween and force tissue fluid to flow within the at least one channel during the sealing process.

The tissue engaging surfaces of the electrodes can be disposed as pairs of longitudinal strips extending from a proximal end of each jaw member to a distal end thereof. At least one traversally oriented channel may be defined between respective tissue engaging surfaces on at least one jaw member.

The tissue engaging surfaces of the electrodes are disposed as series of longitudinal strips extending from a proximal end of each jaw member to a distal end thereof, with the first and second strips of the series being substantially offset relative to one another.

The tissue engaging surfaces of the electrodes are disposed as series of longitudinal strips extending from a proximal end of each jaw member to a distal end thereof, the first, second and third strips of the series being substantially offset relative to one another.

### BRIEF DESCRIPTION OF THE DRAWINGS

Various embodiments of the subject instrument are described herein with reference to the drawings wherein:
FIG. 1A is a perspective view of an endoscopic forceps having an electrode assembly;
FIG. 1B is a perspective view of an open forceps having an electrode assembly;
FIG. 2 is an enlarged, perspective view of the electrode assembly of the forceps of FIG. 1B shown in an open configuration and lacking the longitudinal strips of the present invention;
FIG. 3A is an enlarged, schematic view of an electrode assembly showing a pair of opposing, concentrically-oriented electrodes disposed on a pair of opposing jaw members and lacking the longitudinal strips of the present invention;
FIG. 3B is a partial, side cross-sectional view of the electrode assembly of FIG. 3A;
FIG. 4A is an enlarged, schematic view of an electrode assembly showing a plurality of concentrically-oriented electrode micro-sealing pads disposed on the same jaw member and lacking the longitudinal strips of the present invention;
FIG. 4B is a greatly enlarged view of the area of detail in FIG. 4A showing the electrical path during activation of the electrode assembly;
FIG. 4C is an enlarged schematic view showing the individual micro-sealing sites and viable tissue areas between the two jaw members after activation;
FIG. 5A is a schematic, perspective view of the jaw members lacking the longitudinal strips of the present invention approximating tissue;
FIG. 5B is a schematic, perspective view of the jaw members lacking the longitudinal strips of the present invention grasping tissue; and
FIG. 5C is a schematic, perspective view showing a series of micro-seals disposed in a pattern across the tissue after activation of the electrode assembly.
FIG. 6 is plan view of a tissue seal sealed by an electrosurgical forceps according to the prior art showing a potential failure mechanism due to fluid entry into the seal perimeter;
FIG. 7A is a plan view of one jaw member of an electrosurgical forceps having an electrode with a plurality of slots;
FIG. 7B is a view of a distal end of jaw members of the electrosurgical forceps according to FIG. 7A;
FIG. 8A is a plan view of one jaw member of an electrosurgical forceps having an electrode with a plurality of slots;
FIG. 8B is a view of a distal end of jaw members of the electrosurgical forceps according to FIG. 8A;
FIG. 9A is a perspective view of one jaw member of an electrosurgical forceps having an electrode with a plurality of slots;
FIG. 9B is a view of a distal end of jaw members of the electrosurgical forceps according to FIG. 9A;
FIG. 10A is a plan view of one jaw member of an electrosurgical forceps having an array of individual electrodes ; and
FIG. 10B is an elevation view of an end effector assembly of an electrosurgical forceps having jaw members according to FIG. 10A.

### DETAILED DESCRIPTION

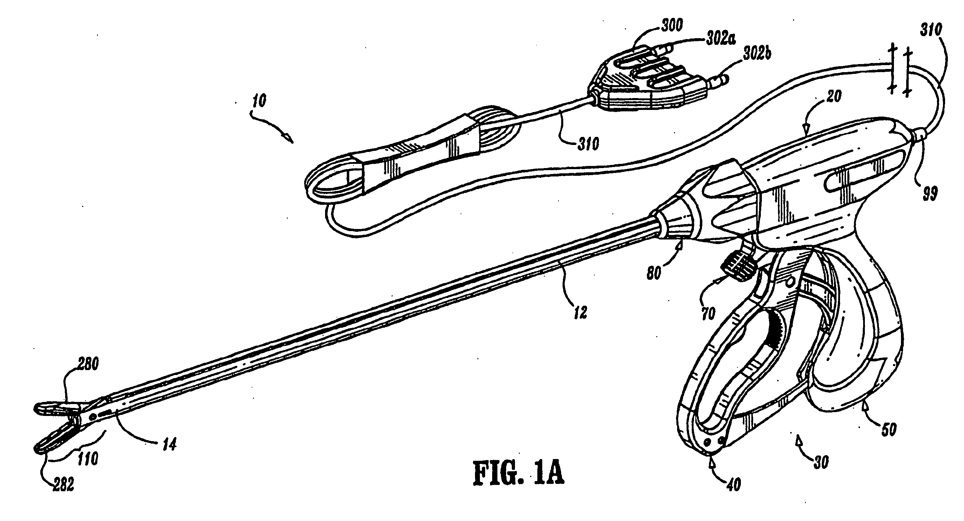

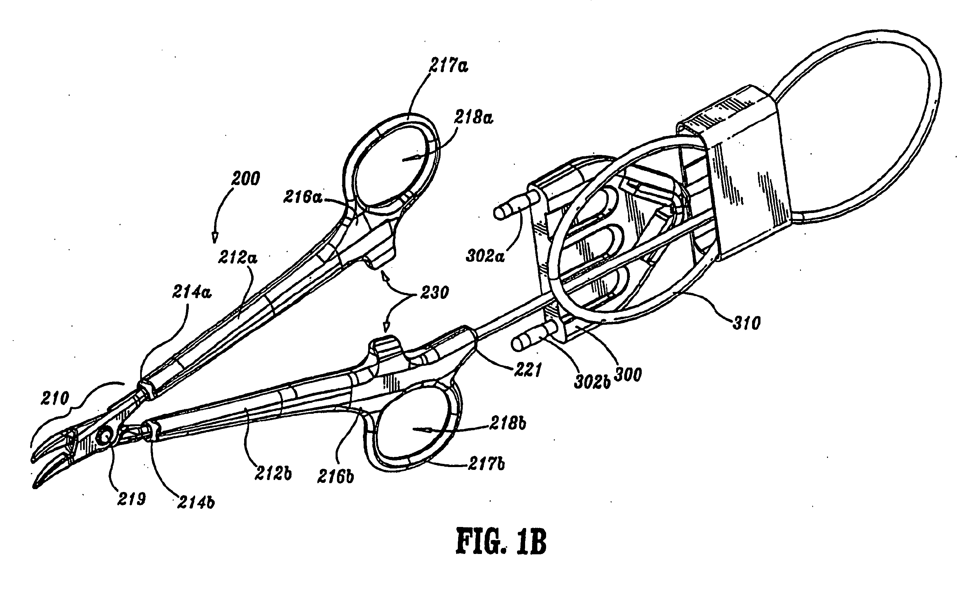

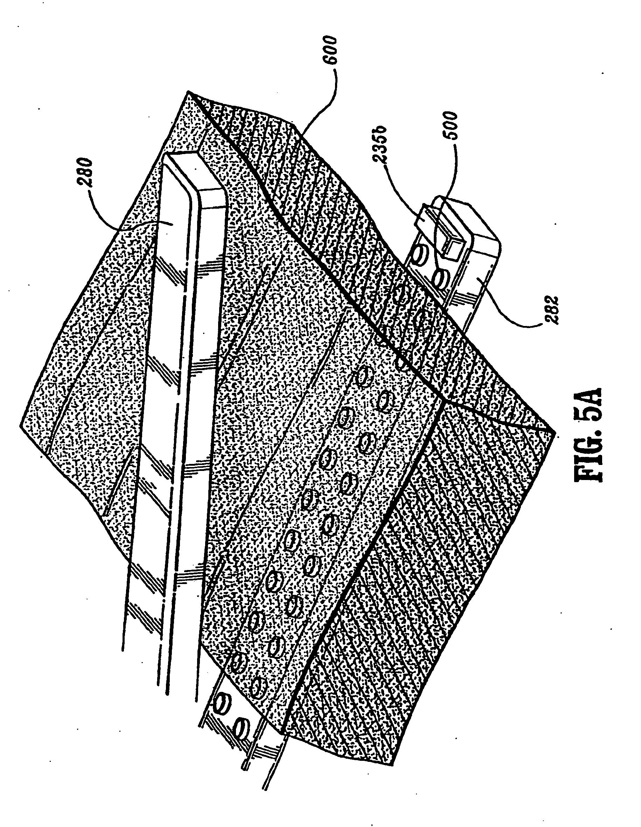

Referring now to FIG. 1A, a bipolar forceps 10 is shown for use with various surgical procedures. Forceps 10 generally includes a housing 20, a handle assembly 30, a rotating assembly 80, an activation assembly 70 and an electrode assembly 110 which mutually cooperate to grasp and seal tissue 600 (See FIGS. 5A-5C). Although the majority of the figure drawings depict a bipolar forceps 10 for use in connection with endoscopic surgical procedures, an open forceps 200 is also contemplated for use in connection with traditional open surgical procedures and is shown by way of example in FIG. 1B and is described below. For the purposes herein, either an endoscopic instrument or an open instrument may be utilized with the electrode assembly described herein. Obviously, different electrical and mechanical connections and considerations apply to each particular type of instrument, however, the novel aspects with respect to the electrode assembly and its operating characteristics remain generally consistent with respect to both the open or endoscopic designs.

More particularly, forceps 10 includes a shaft 12 which has a distal end 14 dimensioned to mechanically engage a jaw assembly 110 and a proximal end 16 which mechanically engages the housing 20. The shaft 12 may be bifurcated at the distal end 14 thereof to receive the jaw assembly 110. The proximal end 16 of shaft 12 mechanically engages the rotating assembly 80 to facilitate rotation of the jaw assembly 110. In the drawings and in the descriptions which follow, the term "proximal", as is traditional, will refer to the end of the forceps 10 which is closer to the user, while the term "distal" will refer to the end which is further from the user.

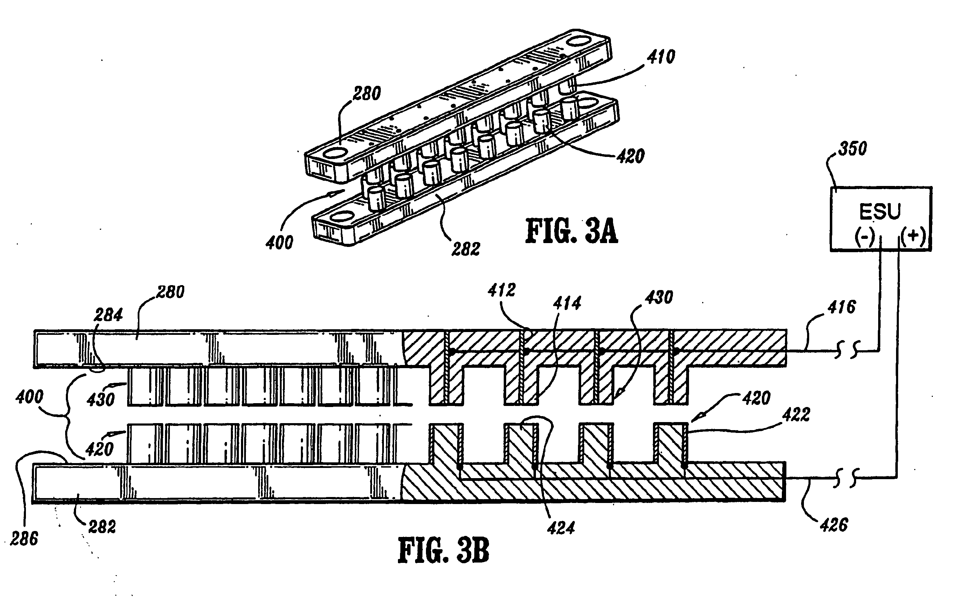

Forceps 10 also includes an electrical interface or plug 300 which connects the forceps 10 to a source of electrosurgical energy, e.g., an electrosurgical generator 350 (See FIG. 3B). Plug 300 includes a pair of prong members 302a and 302b which are dimensioned to mechanically and electrically connect the forceps 10 to the electrosurgical generator 350. An electrical cable 310 extends from the plug 300 to a sleeve 99 which securely connects the cable 310 to the forceps 10. Cable 310 is internally divided within the housing 20 to transmit electrosurgical energy through various electrical feed paths to the jaw assembly 110 as explained in more detail below.

Handle assembly 30 includes a fixed handle 50 and a movable handle 40. Fixed handle 50 is integrally associated with housing 20 and handle 40 is movable relative to fixed handle 50 to actuate a pair of opposing jaw members 280 and 282 of the jaw assembly 110 as explained in more detail below. The activation assembly 70 is selectively movable by the surgeon to energize the jaw assembly 110. Movable handle 40 and activation assembly 70 are typically of unitary construction and are operatively connected to the housing 20 and the fixed handle 50 during the assembly process.

As mentioned above, jaw assembly 110 is attached to the distal end 14 of shaft 12 and includes a pair of opposing jaw members 280 and 282. Movable handle 40 of handle assembly 30 imparts movement of the jaw members 280 and 282 about a pivot pin 119 from an open position wherein the jaw members 280 and 282 are disposed in spaced relation relative to one another for approximating tissue 600, to a clamping or closed position wherein the jaw members 280 and 282 cooperate to grasp tissue 600 therebetween (See FIGS. 5A-5C).

It is envisioned that the forceps 10 may be designed such that it is fully or partially disposable depending upon a particular purpose or to achieve a particular result. For example, jaw assembly 110 may be selectively and releasably engageable with the distal end 14 of the shaft 12 and/or the proximal end 16 of shaft 12 may be selectively and releasably engageable with the housing 20 and the handle assembly 30. In either of these two instances, the forceps 10 would be considered "partially disposable" or "reposable", i.e., a new or different jaw assembly 110 (or jaw assembly 110 and shaft 12) selectively replaces the old jaw assembly 110 as needed.

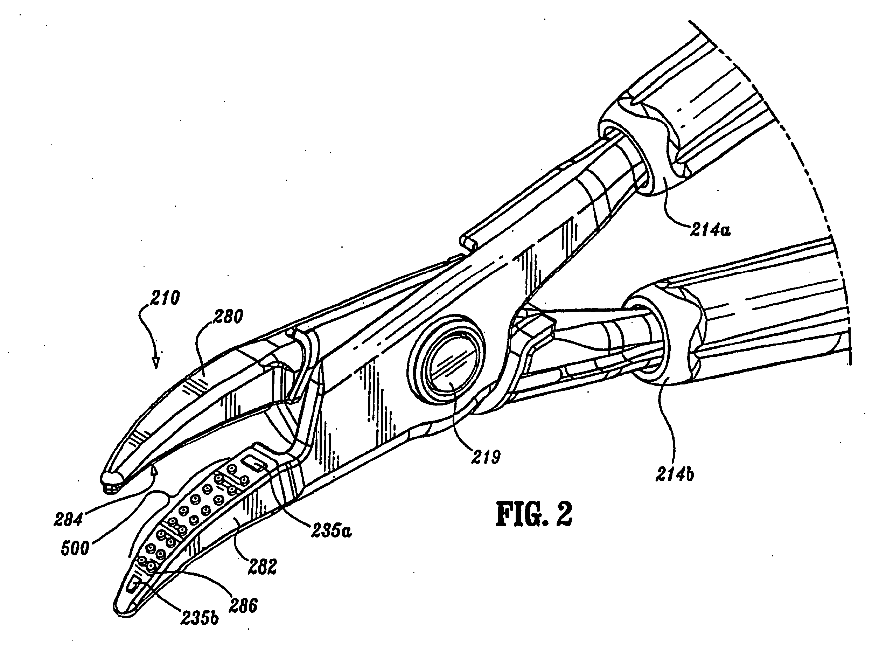

Referring now to FIGS. 1B and 2, an open forceps 200 includes a pair of elongated shaft portions 212a each having a proximal end 216a and 216b, respectively, and a distal end 214a and 214b, respectively. The forceps 200 includes jaw assembly 210 which attaches to distal ends 214a and 214b of shafts 212a and 212b, respectively. Jaw assembly 210 includes opposing jaw members 280 and 282 which are pivotably connected about a pivot pin 219.

Each shaft 212a and 212b includes a handle 217a and 217b disposed at the proximal end 216a and 216b thereof which each define a finger hole 218a and 218b, respectively, therethrough for receiving a finger of the user. As can be appreciated, finger holes 218a and 218b facilitate movement of the shafts 212a and 212b relative to one another which, in turn, pivot the jaw members 280 and 282 from an open position wherein the jaw members 280 and 282 are disposed in spaced relation relative to one another for approximating tissue 600 to a clamping or closed position wherein the jaw members 280 and 282 cooperate to grasp tissue 600 therebetween. A ratchet 230 is included for selectively locking the jaw members 280 and 282 relative to one another at various positions during pivoting.

Each position associated with the cooperating ratchet interfaces 230 holds a specific, i.e., constant, strain energy in the shaft members 212a and 212b which, in turn, transmits a specific closing force to the jaw members 280 and 282. It is envisioned that the ratchet 230 may include graduations or other visual markings which enable the user to easily and quickly ascertain and control the amount of closure force desired between the jaw members 280 and 282.

One of the shafts, e.g., 212b, includes a proximal shaft connector /flange 221 which is designed to connect the forceps 200 to a source of electrosurgical energy such as an electrosurgical generator 350 (FIG. 3B). More particularly, flange 221 mechanically secures electrosurgical cable 310 to the forceps 200 such that the user may selectively apply electrosurgical energy as needed. The proximal end of the cable 310 includes a similar plug 300 as described above with respect to FIG. 1A. The interior of cable 310 houses a pair of leads which conduct different electrical potentials from the electrosurgical generator 350 to the jaw members 280 and 282 as explained below with respect to FIG. 2.

The jaw members 280 and 282 are generally symmetrical and include similar component features which cooperate to permit facile rotation about pivot 219 to effect the grasping of tissue 600. Each jaw member 280 and 282 includes a non-conductive tissue contacting surface 284 and 286, respectively, which cooperate to engage the tissue 600 during treatment.

As best shown in FIG. 2, the various electrical connections of the electrode assembly 210 are typically configured to provide electrical continuity to an array of electrode micro-sealing pads 500 of disposed across one or both jaw members 280 and 282. The electrical paths 416, 426 or 516, 526 from the array of electrode micro-sealing pads 500 are typically mechanically and electrically interfaced with corresponding electrical connections (not shown) disposed within shafts 212a and 212b, respectively. As can be appreciated, these electrical paths 416, 426 or 516, 526 may be permanently soldered to the shafts 212a and 212b during the assembly process of a disposable instrument or, alternatively, selectively removable for use with a reposable instrument.

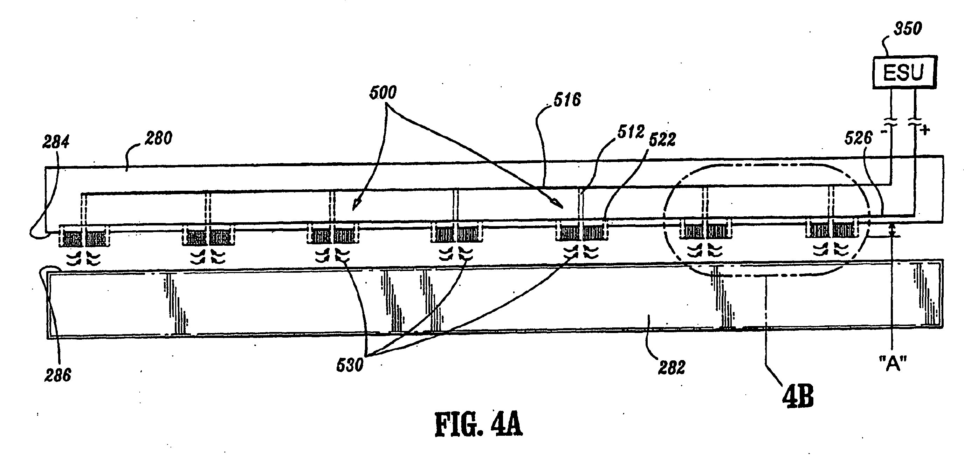

As best shown in FIGS. 4A-4C, the electrical paths are connected to the plurality of electrode micro-sealing pads 500 within the jaw assembly 210. More particularly, the first electrical path 526 (i.e., an electrical path having a first electrical potential) is connected to each ring electrode 522 of each electrode micro-sealing pad 500. The second electrical path 516 (i.e., an electrical path having a second electrical potential) is connected to each post electrode 522 of each electrode micro-sealing pad 500.

The electrical paths 516 and 526 typically do not encumber the movement of the jaw members 280 and 282 relative to one another during the manipulation and grasping of tissue 400. Likewise, the movement of the jaw members 280 and 282 do not unnecessarily strain the electrical paths 516 and 526 or their respective connections 517, 527.

As best seen in FIGS. 2-5C, jaw members 280 and 282 both include non-conductive tissue contacting surfaces 284 and 286, respectively, disposed along substantially the entire longitudinal length thereof (i.e., extending substantially from the proximal to distal end of each respective jaw member 280 and 284). The non-conductive tissue contacting surfaces 284 and 286 may be made from an insulative material such as ceramic due to its hardness and inherent ability to withstand high temperature fluctuations. Alternatively, the non-conductive tissue contacting surfaces 284 and 286 may be made from a material or a combination of materials having a high Comparative Tracking Index (CTI) in the range of about 300 to about 600 volts. Examples of high CTI materials include nylons and syndiotactic polystryrenes such as QUESTRA^{®} manufactured by DOW Chemical. Other materials may also be utilized either alone or in combination, e.g., Nylons, Syndiotactic-polystryrene (SPS), Polybutylene Terephthalate (PBT), Polycarbonate (PC), Acrylonitrile Butadiene Styrene (ABS), Polyphthalamide (PPA), Polymide, Polyethylene Terephthalate (PET), Polyamide-imide (PAI), Acrylic (PMMA), Polystyrene (PS and HIPS), Polyether Sulfone (PES), Aliphatic Polyketone, Acetal (POM) Copolymer, Polyurethane (PU and TPU), Nylon with Polyphenylene-oxide dispersion and Acrylonitrile Styrene Acrylate. Typically, the non-conductive tissue contacting surfaces 284 and 286 are dimensioned to securingly engage and grasp the tissue 600 and may include serrations (not shown) or roughened surfaces to facilitate approximating and grasping tissue.

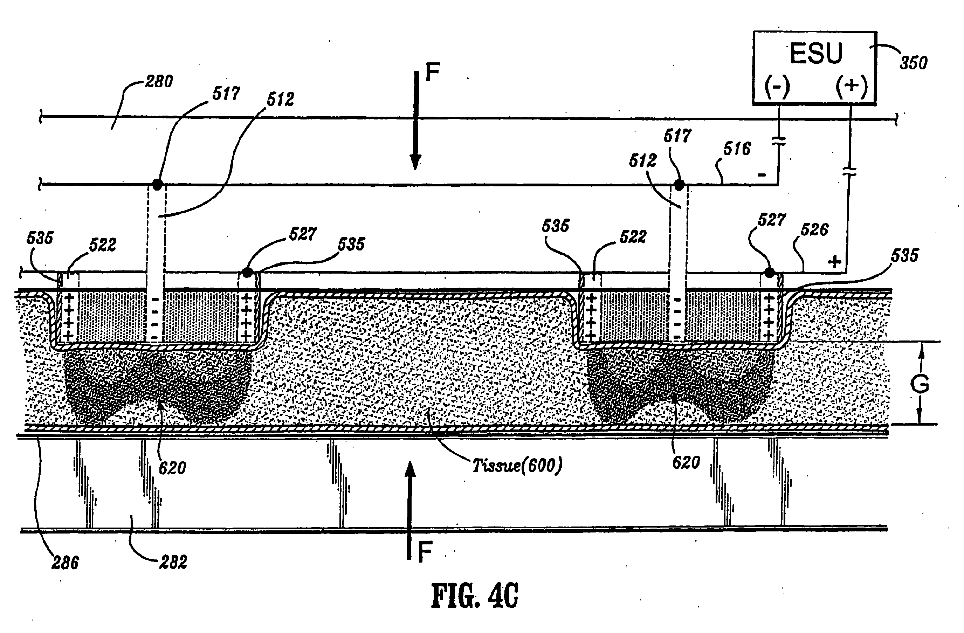

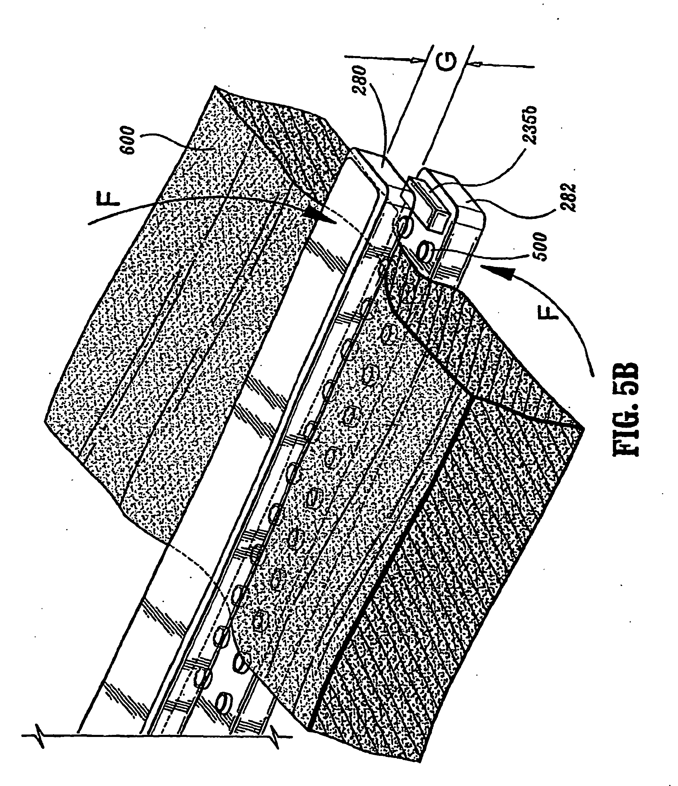

It is envisioned that one of the jaw members, e.g., 282, includes at least one stop member 235a, 235b (FIG. 2) disposed on the inner facing surface of the sealing surfaces 286. Alternatively or in addition, one or more stop members 235a, 235b may be positioned adjacent to the non-conductive sealing surfaces 284, 286 or proximate the pivot 219. The stop members 235a, 235b are typically designed to define a gap "G" (FIG. 5B) between opposing jaw members 280 and 282 during the micro-sealing process. The separation distance during micro-sealing or the gap distance "G" is within the range of about 0.001 inches (-0.03 millimeters) to about 0.006 inches (-0.016 millimeters). One or more stop members 235a, 235b may be positioned on the distal end and proximal end of one or both of the jaw members 280, 282 or may be positioned between adjacent electrode micro-sealing pads 500. Moreover, the stop members 235a and 235b may be integrally associated with the non-conductive tissue contacting surfaces 284 and 286. It is envisioned that the array of electrode micro-sealing pads 500 may also act as stop members for regulating the distance "G" between opposing jaw members 280, 282 (See FIG. 4C).

As mentioned above, the effectiveness of the resulting micro-seal is dependent upon the pressure applied between opposing jaw members 280 and 282, the pressure applied by each electrode micro-sealing pad 500 at each micro-sealing site 620 (FIG. 4C), the gap "G" between the opposing jaw members 280 and 282 (either regaled by a stop member 235a, 235b or the array of electrode micro-sealing pads 500) and the control of the electrosurgical intensity during the micro-sealing process. Applying the correct force is important to oppose the walls of the tissue; to reduce the tissue impedance to a low enough value that allows enough current through the tissue; and to overcome the forces of expansion during tissue heating in addition to contributing towards creating the required end tissue thickness which is an indication of a good micro-seal. Regulating the gap distance and regulating the electrosurgical intensity ensure a consistent seal quality and reduce the likelihood of collateral damage to surrounding tissue.

As best shown in FIG. 2, the electrode micro-sealing pads 500 are arranged in a longitudinal, pair-like fashion along the tissue contacting surfaces 286 and/or 284. Two or more micro-sealing pads 500 may extend transversally across the tissue contacting surface 286. FIGS. 3A and 3B show one embodiment of the present disclosure wherein the electrode micro-sealing pads 500 include a ring electrode 422 disposed on one jaw members 282 and a post electrode 412 disposed on the other jaw member 280. The ring electrode 422 includes an insulating material 424 disposed therein to form a ring electrode and insulator assembly 420 and the post electrode 422 includes an insulating material disposed therearound to form a post electrode and insulator assembly 430. Each post electrode assembly 430 and the ring electrode assembly 420 of this embodiment together define one electrode micro-sealing pad 400. Although shown as a circular-shape, ring electrode 422 may assume any other annular or enclosed configuration or alternatively partially enclosed configuration such as a C-shape arrangement.

As best shown in FIG. 3B, the post electrode 422 is concentrically centered opposite the ring electrode 422 such that when the jaw members 280 and 282 are closed about the tissue 600, electrosurgical energy flows from the ring electrode 422, through tissue 600 and to the post electrode 412. The insulating materials 414 and 424 isolate the electrodes 412 and 422 and prevent stray current tracking to surrounding tissue. Alternatively, the electrosurgical energy may flow from the post electrode 412 to the ring electrode 422 depending upon a particular purpose.

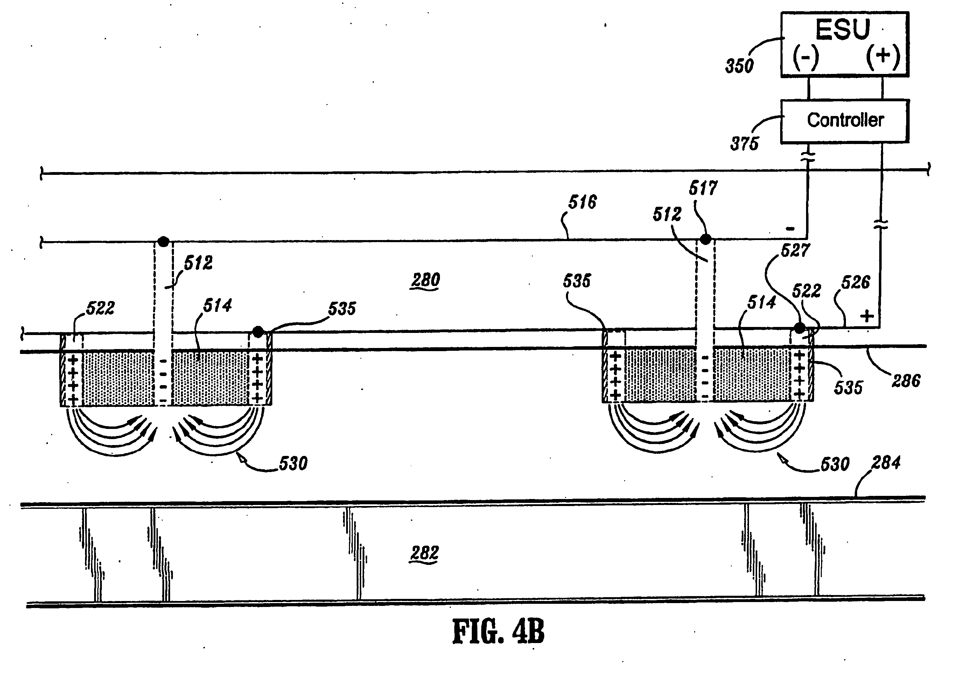

FIGS. 4A-4C show jaw assembly 210 for micro-sealing tissue 600 wherein each electrode micro-sealing pad 500 is disposed on a single jaw member, e.g., jaw member 280. More particularly and as best illustrated in FIG. 4B, each electrode micro-sealing pad 500 consists of an inner post electrode 512 which is surrounded by an insulative material 514, e.g., ceramic. The insulative material 514 is, in turn, encapsulated by a ring electrode 522. A second insulative material 535 (or the same insulative material 514) may be configured to encase the ring electrode 522 to prevent stray electrical currents to surrounding tissue.

The ring electrode 522 is connected to the electrosurgical generator 350 by way of a cable 526 (or other conductive path) which transmits a first electrical potential to each ring electrode 522 at connection 527. The post electrode 512 is connected to the electrosurgical generator 350 by way of a cable 516 (or other conductive path) which transmits a second electrical potential to each post electrode 522 at connection 517. A controller 375 (See FIG. 4B) may be electrically interposed between the generator 350 and the electrodes 512, 522 to regulate the electrosurgical energy supplied thereto depending upon certain electrical parameters, current impedance, temperature, voltage, etc. For example, the instrument or the controller may include one or more smart sensors (not shown) which communicate with the electrosurgical generator 350 (or smart circuit, computer, feedback loop, etc.) to automatically regulate the electrosurgical intensity (waveform, current, voltage, etc.) to enhance the micro-sealing process. The sensor may measure or monitor one or more of the following parameters: tissue temperature, tissue impedance at the micro-seal, change in impedance of the tissue over time and/or changes in the power or current applied to the tissue over time. An audible or visual feedback monitor (not shown) may be employed to convey information to the surgeon regarding the overall micro-seal quality or the completion of an effective tissue micro-seal.

Moreover, a PCB circuit of flex circuit (not shown) may be utilized to provide information relating to the gap distance (e.g., a proximity detector may be employed) between the two jaw members 280 and 282, the micro-sealing pressure between the jaw members 280 and 282 prior to and during activation, load (e.g., strain gauge may be employed), the tissue thickness prior to or during activation, the impedance across the tissue during activation, the temperature during activation, the rate of tissue expansion during activation and micro-sealing. It is envisioned that the PCB circuit may be designed to provide electrical feedback to the generator 350 rotating to one or more of the above parameters either on a continuous basis or upon inquiry from the generator 350. For example, a PCB circuit may be employed to control the power, current and/or type of current waveform from the generator 350 to the jaw members 280, 282 to reduce collateral damage to surrounding tissue during activation, e.g., thermal spread, tissue vaporization and/or steam from the treatment site. Examples of a various control circuits, generators and algorithms which may be utilized are disclosed in U.S. Patent No 6,228,080 and U.S. Application Serial No. 10/073,761.

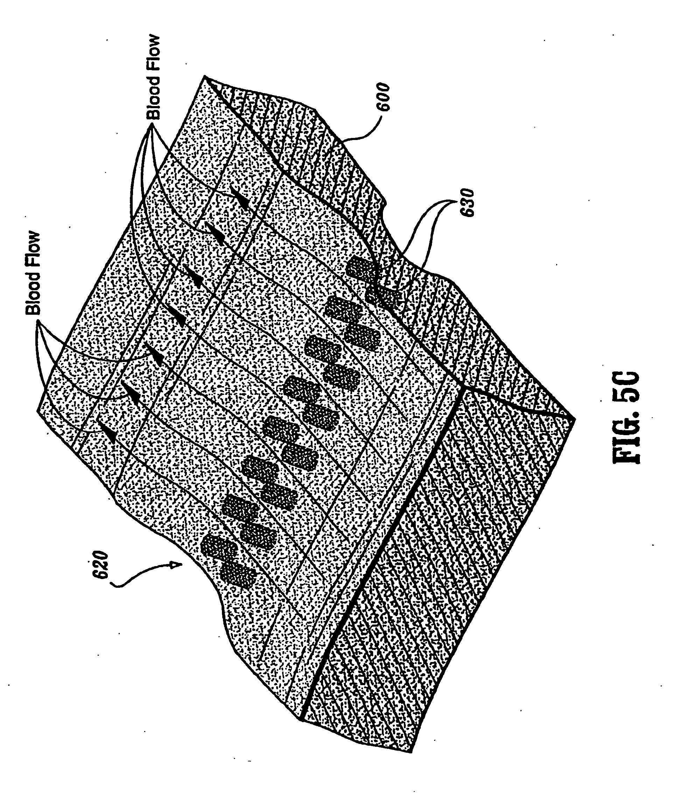

In use as depicted in FIGS. 5A-5C, the surgeon initially approximates the tissue (FIG. 5A) between the opposing jaw member 280 and 282 and then grasps the tissue 600 (FIG. 5B) by actuating the jaw members 280, 282 to rotate about pivot 219. Once the tissue is grasped, the surgeon selectively activates the generator 350 to supply electrosurgical energy to the array of the electrode micro-sealing pads 500. More particularly, electrosurgical energy flows from the ring electrode 522, through the tissue 600 and to the post electrode 512 (See FIGS. 4B and 4C). As a result thereof, an intermittent pattern of individual micro-seals 630 is created along and across the tissue 600 (See FIG. 5C). The arrangement of the micro-sealing pads 500 across the tissue only seals the tissue which is between each micro-sealing pad 500 and the opposing jaw member 282. The adjacent tissue remains viable which, as can be appreciated, allows blood and nutrients to flow through the sealing site 620 and between the individual micro-seals 630 to promote tissue healing and reduce the chances of tissue necrosis. By selectively regulating the closure pressure "F", gap distance "G", and electrosurgical intensity, effective and consistent micro-seals 630 may be created for many different tissue types.

It is further envisioned that selective ring electrodes and post electrodes may have varying electric potentials upon activation. For example, at or proximate the distal tip of one of the jaw members, one or a series of electrodes may be electrically connected to a first potential and the corresponding electrodes (either on the same jaw or perhaps the opposing jaw) may be connected to a second potential. Towards the proximal end of the jaw member, one or a series of electrodes may be connected to a third potential and the corresponding electrodes connected to yet a fourth potential. As can be appreciated, this would allow different types of tissue sealing to take place at different portions of the jaw members upon activation. For example, the type of sealing could be based upon the type of tissues involved or perhaps the thickness of the tissue. To seal larger tissue, the user would grasp the tissue more towards the proximal portion of the opposing jaw members and to seal smaller tissue, the user would grasp the tissue more towards the distal portion of the jaw members. It is also envisioned that the pattern and/or density of the micro-sealing pads may be configured to seal different types of tissue or thicknesses of tissue along the same jaw members depending upon where the tissue is grasped between opposing jaw members.

For example, it is envisioned that by making the forceps 100, 200 disposable, the forceps 100, 200 is less likely to become damaged since it is only intended for a single use and, therefore, does not require cleaning or sterilization. As a result, the functionality and consistency of the vital micro-sealing components, e.g., the conductive micro-sealing electrode pads 500, the stop member(s) 235a, 235b, and the insulative materials 514, 535 will assure a uniform and quality seal.

Experimental results suggest that the magnitude of pressure exerted on the tissue by the micro-sealing pads 112 and 122 is important in assuring a proper surgical outcome, maintaining tissue viability. Tissue pressures within a working range of about 3 kg/cm² to about 16 kg/cm² and, more particularly, within a working range of 7 kg/cm² to 13 kg/cm² have been shown to be effective for micro-sealing various tissue types and vascular bundles.

The shafts 212a and 212b can be manufactured such that the spring constant of the shafts 212a and 212b, in conjunction with the placement of the interfacing surfaces of the ratchet 230, will yield pressures within the above working range. In addition, the successive positions of the ratchet interfaces increase the pressure between opposing micro-sealing surfaces incrementally within the above working range.

It is envisioned that the outer surface of the jaw members 280 and 282 may include a nickel-based material or coating which is designed to reduce adhesion between the jaw members 280, 282 (or components thereof) with the surrounding tissue during activation and micro-sealing. Moreover, it is also contemplated that other components such as the shaft portions 212a, 212b and the rings 217a, 217b may also be coated with the same or a different "non-stick" material. Typically, the non-stick materials are of a class of materials that provide a smooth surface to prevent mechanical tooth adhesions.

It is also contemplated that the tissue contacting portions of the electrodes and other portions of the micro-sealing pads 400, 500 may also be made from or coated with non-stick materials. When utilized on these tissue contacting surfaces, the non-stick materials provide an optimal surface energy for eliminating sticking due in part to surface texture and susceptibility to surface breakdown due electrical effects and corrosion in the presence of biologic tissues. It is envisioned that these materials exhibit superior non-stick qualities over stainless steel and should be utilized in areas where the exposure to pressure and electrosurgical energy can create localized "hot spots" more susceptible to tissue adhesion. As can be appreciated, reducing the amount that the tissue "sticks" during micro-sealing improves the overall efficacy of the instrument.

The non-stick materials may be manufactured from one (or a combination of one or more) of the following "non-stick" materials: nickelchrome, chromium nitride, MedCoat 2000 manufactured by The Electrolizing Corporation of OHIO, Inconel 600 and tin-nickel. Inconel 600 coating is a so-called "super alloy" which is manufactured by Special Metals, Inc. located in Conroe Texas. The alloy is primarily used in environments which require resistance to corrosion and heat. The high Nickel content of Inconel 600 makes the material especially resistant to organic corrosion. As can be appreciated, these properties are desirable for bipolar electrosurgical instruments which are naturally exposed to high temperatures, high RF energy and organic matter. Moreover, the resistivity of Inconel 600 is typically higher than the base electrode material which further enhances desiccation and micro-seal quality.

One particular class of materials disclosed herein has demonstrated superior non-stick properties and, in some instances, superior micro-seal quality. For example, nitride coatings which include, but not are not limited to: TiN, ZrN, TiAlN, and CrN are preferred materials used for non-stick purposes. CrN has been found to be particularly useful for non-stick purposes due to its overall surface properties and optimal performance. Other classes of materials have also been found to reducing overall sticking. For example, high nickel/chrome alloys with a Ni/Cr ratio of approximately 5:1 have been found to significantly reduce sticking in bipolar instrumentation.

It is also envisioned that the micro-sealing pads 400, 500 may be arranged in many different configurations across or along the jaw members 280, 282 depending upon a particular purpose. Moreover, it is also contemplated that a knife or cutting element (not shown) may be employed to sever the tissue 600 between a series of micro-sealing pads 400, 500 depending upon a particular purpose. The cutting element may include a cutting edge to simply mechanically cut tissue 600 and/or may be configured to electrosurgically cut tissue 600.

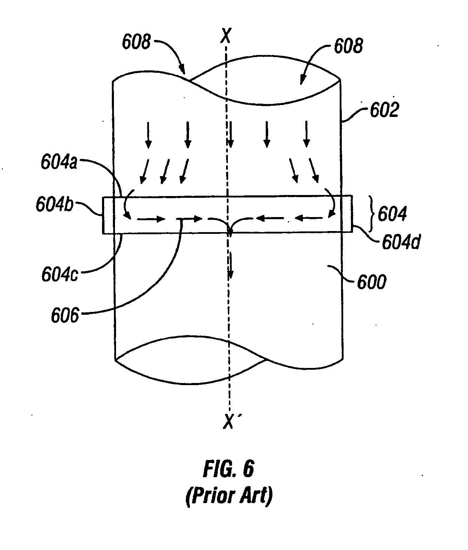

FIG. 6 discloses a resulting tissue seal sealed by an electrosurgical forceps according to the prior art showing a potentially weaker seal area due to entry of fluid into the seal perimeter during sealing. More particularly, tissue 600 of a lumen 602 of a patient's body such as the large or small intestines or any other passage or vessel is subject to a tissue seal 604 performed by an electrosurgical forceps of the prior art (not shown). The tissue seal 604 is typically formed utilizing radiofrequency (RF) energy. The lumen 602 has an approximate' centerline axis X-X'. The seal 604 has a perimeter generally of four contiguous sides 604a, 604b, 604c and 604d and a central portion 606. Two sides 604a and 604c extend in a direction generally orthogonal to the centerline axis X-X' of the lumen 602 and parallel to each other, while the two sides 604b and 604d extend in a direction generally parallel to the centerline axis X-X'. It has been determined that during sealing, fluid 608 may enter at a side of the perimeter such as side 604a and propagate to the central portion 606 of the tissue seal 604. A weaker seal may develop as a result of increased fluid in a particular tissue area.

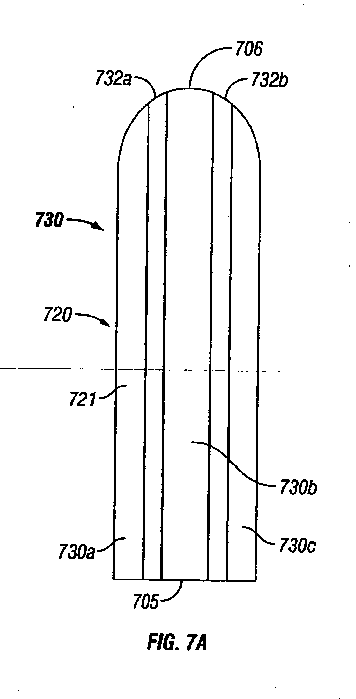

FIG. 7A illustrates a jaw member 720 of an electrode assembly 700 for use with an electrosurgical forceps which includes an electrode 721 with a plurality of slots or channels 732a and 732b. More particularly, electrode 721 of jaw member 722 of electrode assembly 700 includes a substantially longitudinal, planar, tissue engaging surface 730 which has at least first channel 732a, and typically includes a second channel 732b. Each channel 732a and 732b is disposed in a lengthwise direction from a proximal end 705 to a distal end 706 of the electrode 721 so as to divide surface 730 into at least two substantially longitudinal surfaces 730a and 730c. A third substantially longitudinal surface 730b is disposed between channels 732a and 732c.

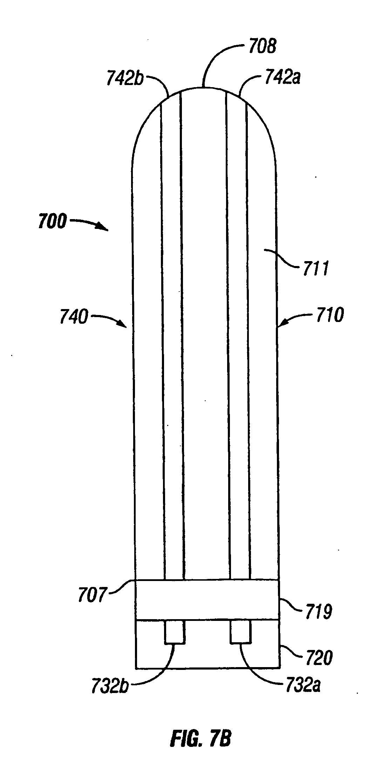

FIG. 7B shows upper jaw member 710 of electrode assembly 700. More particularly, upper jaw member 710 is similar to jaw member 720 and includes a corresponding electrode member 711 which has a substantially longitudinal, planar, tissue engaging surface 740. Jaw members 710 and 720 are pivotably connected around a pivot pin 719, and are movable from an open position wherein the jaw members 710 and 720 are disposed in spaced relation relative to one another for manipulating tissue 600, to a clamping or closed position wherein the jaw members 710 and 720 cooperate to grasp tissue 600 therebetween. Jaw members 710 and 720 operate in an analogous manner as described previously with respect to jaw members 280 and 282 (See FIGS. 5A-5C).

Surface 740 includes at least a first channel 742a and typically includes a second channel 742b. Each channel 742a and 742b is disposed in a lengthwise direction from a proximal end 705 to a distal end 706 of the electrode 710 so as to divide surface 740 into surfaces 740a, 740b, and 740c. Surface 730 of jaw member 720 and surface 740 of jaw member 710 are configured so that channels 742a and 742b substantially correspond to channels 732a and 732b, and consequently, so that the surfaces 730a, 730b and 730c substantially correspond with or are in vertical registration with surfaces 740a, 740b and 740c.

The corresponding or counterpart channels 732a and 742a, and the corresponding or counterpart channels 732b and 742b form a plurality of corresponding or counterpart electrode surfaces 730a and 740a, 730b and 740b, and 730c and 740c which form tissue seals characterized by potential tissue fluid flow paths. It is envisioned that arranging the electrodes 711 and 721 in this fashion will impede the flow of tissue fluid during the sealing process which allows a stronger seal to develop. In other words, the envisioned electrode 711 and 721 arrangement with channels 732a-732c and 742a-742c inhibits the flow of fluid through the tissue seal, thereby increasing the structural integrity of the tissue seal and decreasing the probability of tissue seal rupture.

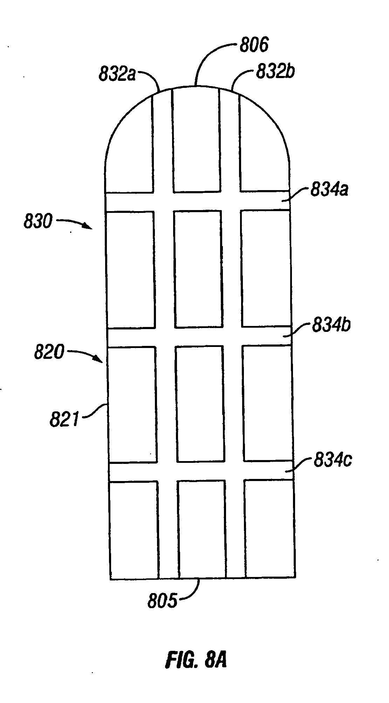

FIG. 8A illustrates a jaw member 820 of an electrosurgical forceps having an electrode arrangement. More particularly, an electrode 821 of jaw member 820 of an electrode assembly 800 includes a substantially longitudinal, planar, tissue engaging electrode surface 830 which has a plurality of longitudinal and transverse or traversally oriented channels 832a and 832b and 834a to 834c, respectively, which extend lengthwise from proximal end 805 to distal end 806 and across the jaw member 820.

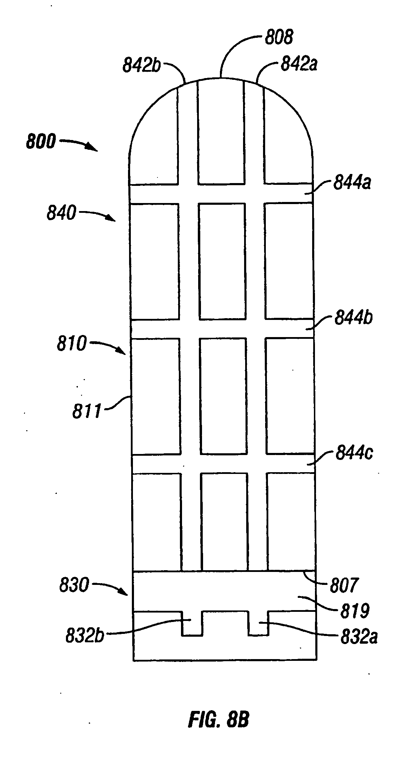

Referring to FIG. 8B, jaw member 810 includes or is characterized by a similar arrangement. An electrode 811 of jaw member 810 of electrode assembly 800 has a substantially longitudinal, planar tissue engaging surface 840 which includes longitudinal channels 842a and 842b and transverse channels 844a to 844c.

Jaw member 810 and jaw member 820 are pivotably connected around pivot pin 819 such that jaw members 810 and 820 are movable from an open position wherein the jaw members 810 and 820 are disposed in spaced relation relative to one another for manipulating tissue 600, to a clamping or closed position wherein the jaw members 810 and 820 cooperate to grasp tissue 600 therebetween in a similar manner to jaw members 280 and 282 (see FIGS. 5A-5C).

Much like the electrode arrangement of FIGS. 7A and 7B, the electrode tissue engaging surface pattern and channels of each jaw member 810 and 820 are arranged to complement each other to produce a uniform and effective seal. It is envisioned that the fluid path during sealing will be impeded such that a uniform, reliable and effective seal will develop upon activation of the electrodes 811 and 821.

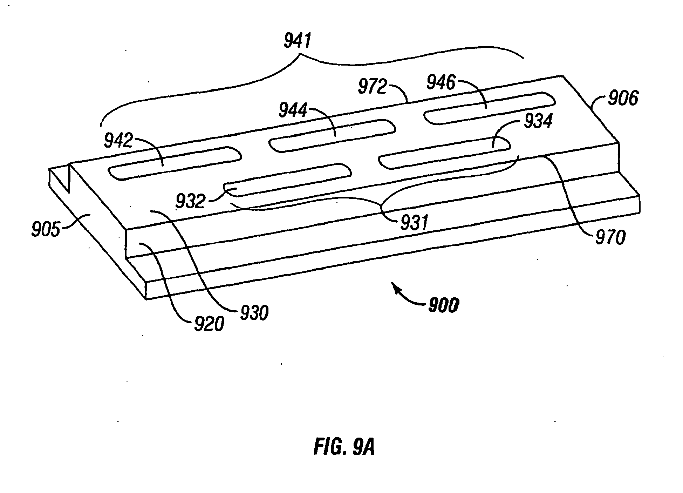

FIG. 9A illustrates a jaw member 920 of an electrosurgical forceps. More particularly, an electrode 921 of jaw member 920 of an electrode assembly 900 has a substantially longitudinal, planar, tissue engaging electrode surface 930. The electrode 921 includes a proximal end 905 and a distal end 906 and is bounded by first and second lateral side edges 970 and 972, respectively. The surface 930 includes a first group 931 of substantially longitudinal slots 932 and 934 aligned in a column oriented from the proximal end 905 to the distal end 906. In one embodiment, the surface 930 includes a second group 941 of substantially longitudinal slots 942, 944 and 946 aligned in a column oriented from the proximal end 905 to the distal end 906. The first group 931 and the second group 941 are disposed on the jaw surface 930 such that the slots 932 and 934 are staggered with respect to the slots 942, 944 and 946.

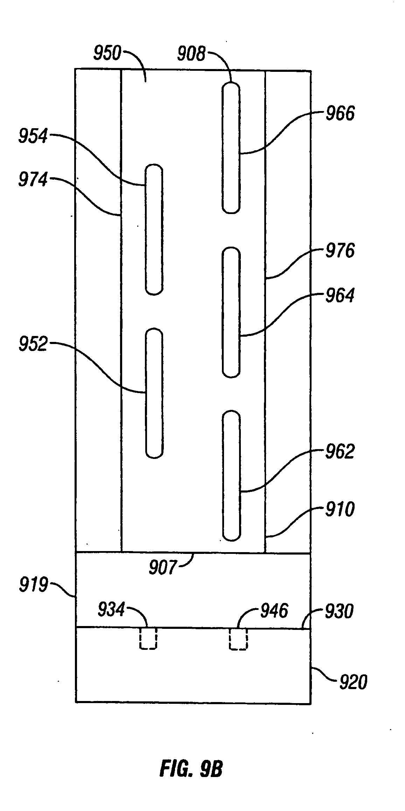

Referring to FIG. 9B, jaw member 910 includes or is characterized by a similar arrangement. An electrode 911 of jaw member 910 of an electrode assembly 900 has a substantially longitudinal, planar, tissue engaging electrode surface 950 which includes a first group 951 of substantially longitudinal slots 952 and 954 aligned in a column oriented from a proximal end 907 to a distal end 908. The electrode 911 is bounded by lateral side edges 974 and 976. The surface 950 may include a second group 961 of substantially longitudinal slots 962, 964 and 966 aligned in a column oriented from the proximal end 907 to the distal end 908. The first group 951 and the second group 961 are disposed on the jaw surface 950 such that the slots 952 and 954 are staggered with respect to the slots 962, 964 and 966. Furthermore, the first group 931 corresponds with or is in vertical registration with first group 951. Similarly, the second group 941 corresponds with or is in vertical registration with second group 961.

Jaw member 910 and jaw member 920 are pivotably connected around pivot pin 919 such that jaw members 910 and 920 are movable from an open position wherein the jaw members 910 and 920 are disposed in spaced relation relative to one another for manipulating tissue 600, to a clamping or closed position wherein the jaw members 910 and 920 cooperate to grasp tissue 600 therebetween in a similar manner to jaw members 280 and 282 (see FIGS. 5A-5C).

Yet again, the staggered slot arrangement forms a tissue seal characterized by a plurality of potential flow paths. Much like the electrode arrangements of FIGS. 7A and 7B, and 8A and 8B, the electrode tissue-engaging surface patterns and channels of each jaw member 910 and 920 are arranged to complement each other to produce a uniform and effective seal. It is envisioned that the fluid path during sealing will be impeded such that a uniform, reliable and effective seal will develop upon activation of the electrodes 911 and 921.

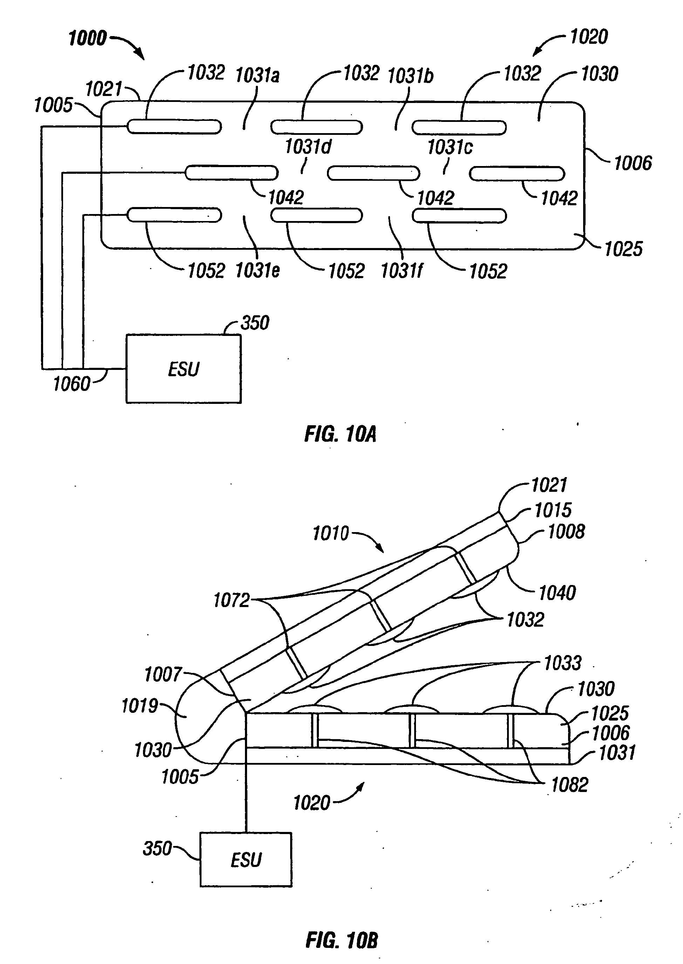

FIGS. 10A and 10B show another example of an electrode arrangement across the surface of a jaw member 1020. More particularly, electrode 1021 includes one or more arrays of tissue-engaging surfaces 1032, 1042 and 1052 which are patterned across the jaw surface 1030 to impede fluid flow during activation which is believed to result in a stronger and more reliable seal. In the particular tissue-engaging surface arrangement of FIGS. 10A and 10B, a similar pattern is envisioned wherein arrays 1032, 1042 and 1052 are disposed within groups to define slots or flow restricting areas 1031 a through 1031f similar to previously described FIGS. 9A and 9B above. Jaw housing 1030 is made typically from an electrically and thermally insulating material such as a temperature resistant plastic or a ceramic or a cool polymer which thermally conducts heat but which is an electrical insulator. Housing 1030 includes an inwardly facing surface 1025 which supports the various arrays of tissue engaging surfaces 1032, 1042 and 1052.

The arrays 1032, 1042 and 1052 are staggered along the length and width of the jaw surface 1025 with respect to one another. It is believed that this electrode arrangement will further impede fluid flow during electrode activation by forcing fluid flow to occur substantially around the electrodes and substantially through slots or flow restricting areas 1031a through 1031f between the array of surfaces 1032, 1042 and 1052, resulting in a more reliable seal. It is also envisioned that other staggered patterns with a greater or lesser number of surface arrays may be employed to strengthen a tissue seal depending upon a particular tissue type.

With particular respect to FIG. 10A, the tissue-engaging surfaces within the arrays 1032, 1042, and 1052 are arranged such that the electrode 1021 carries an electrical potential from generator 350 through lead or leads 1060 to tissue upon electrical activation. It is also envisioned that each tissue-engaging surface of each array of tissue-engaging surfaces may be individually connected to the generator 350.

FIG. 10B shows opposing arrays of tissue-engaging surfaces 1032 and 1033 of jaw members 1020 and 1010, respectively, each connected to a corresponding common element, e.g., conductive electrodes or plates 1021 and 1031, respectively. Each conductive plate 1021 and 1031 carries a different electrical potential through a series of conductive connections 1072 and 1082 to each respective array 1032 and 1033. As can be appreciated, it is envisioned that arranging the arrays in this fashion facilitates manufacturing such that arrays 1032 and 1033 and conductive plates 1021 and 1031 may be held in a die or support tool which the outer housings 1030 and 1040 are overmolded.

The jaw members 1010 and 1020, which are pivotably connected at or in the vicinity of their proximal ends 1005 and 1007 around a pivot pin 1019, from an open position wherein the jaw members 1010 and 1020 are disposed in spaced relation relative to one another for approximating tissue 600, to a clamping or closed position wherein the jaw members 1010 and 1020 cooperate to grasp tissue 600 therebetween in a similar manner to jaw members 280 and 282 (see FIGS. 5A-5C).

It is envisioned that the tissue engaging surfaces 730, 830, 930, 1030 and 740, 840, 940 and 1040 of the electrodes are disposed as a series of longitudinal strips extending from a proximal end of each jaw member to a distal end thereof, the first and second strips being substantially offset relative to one another.

It is also contemplated that the various aforedescribed electrode arrangements may be configured for use with either an open forceps as shown in FIG. 1B or an endoscopic forceps as shown in FIG. 1A. One skilled in the art would recognize that different but known electrical and mechanical considerations would be necessary and apparent to convert an open instrument to an endoscopic instrument to accomplish the same purposes as described herein.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of preferred embodiments. Those skilled in the art will envision other modifications within the scope of the claims appended hereto.

## Claims

1. A bipolar electrosurgical forceps (10), comprising:
first and second opposing jaw members (280, 282, 1010, 1020) having respective tissue engaging surfaces (1032, 1042, 1052, 1033) associated therewith, the first and second jaw members (280, 282, 1010, 1020) adapted for relative movement between an open position to receive tissue and a closed position engaging tissue between said tissue engaging surfaces (1032, 1042, 1052, 1033) to effect a tissue seal upon activation of the forceps (10);
the first and second jaw members (280, 282, 1010, 1020) each including an electrode (1021, 1031) having a plurality of tissue engaging surfaces (1032, 1042, 1052, 1033) which define at least one channel therebetween,
the plurality of tissue engaging surfaces (1032, 1042, 1052) of the first jaw member (280, 1010) being substantially aligned with the plurality of tissue engaging surfaces (1033) of the second jaw member (282, 1020) so as to impede fluid flow therebetween and force tissue fluid to flow within the at least one channel during the sealing process, wherein the bipolar electrocsurgical forceps (10) is **characterized in that**
the tissue engaging surfaces (1032, 1042, 1052, 1033) of the electrodes (1021, 1031) are disposed as a series of longitudinal strips extending from a proximal end of each jaw member (280, 282, 1010, 1020) to a distal end thereof, the first, second and third strips of the series being substantially offset relative to one another.

2. A bipolar electrosurgical forceps (10) according to claim 1, wherein the tissue engaging surfaces (1032, 1042, 1052, 1033) of the electrodes (1021, 1031) are disposed as pairs of longitudinal strips extending from a proximal end of each jaw member (280, 282, 1010, 1020) to a distal end thereof.

3. A bipolar electrosurgical forceps (10) according to claim 2, wherein at least one traversally oriented channel is defined between respective tissue engaging surfaces (1032, 1042, 1052, 1033) on at least one jaw member (280, 282, 1010, 1020).

4. A bipolar electrosurgical forceps (10) according to any one of the preceding claims, wherein each tissue engaging surface (1032, 1042, 1052, 1033) of the electrode (1021, 1031) of the first or second jaw members (280, 282, 1010, 1020) is individually connected to a generator (350).

## Patentansprüche

1. Bipolare elektrochirurgische Zange (10), mit:
einem ersten und einem zweiten gegenüberliegenden Backenelement (280, 282, 1010, 1020), die jeweilige mit ihnen zusammenhängende Gewebeeingriffsflächen (1032, 1042, 1052, 1033) aufweisen, wobei das erste und zweite Backenelement (280, 282, 1010, 1020) für eine relative Bewegung zwischen einer offenen Position, um Gewebe aufzunehmen, und einer geschlossenen Position, die Gewebe zwischen den Gewebeeingriffsflächen (1032, 1042, 1052, 1033) in Eingriff bringt, angepasst ist, um eine Gewebeversiegelung bei Aktivierung der Zange (10) zu bewirken;
wobei das erste und zweite Backenelement (280, 282, 1010, 1020) jeweils eine Elektrode (1021, 1031) mit einer Vielzahl von Gewebeeingriffsflächen (1032, 1042, 1052, 1033) aufweist, die zwischen sich mindestens einen Kanal definieren,
die Vielzahl von Gewebeeingriffsflächen (1032, 1042, 1052) des ersten Backenelements (280, 1010) im Wesentlichen mit der Vielzahl von Gewebeeingriffsflächen (1033) des zweiten Backenelements (282, 1020) ausgerichtet sind, um einen Flüssigkeitsstrom zwischen ihnen zu erschweren und Gewebeflüssigkeit zu zwingen, während des Versiegelungsprozesses in dem mindestens einen Kanal zu fließen, wobei die bipolare elektrochirurgische Zange (10) **dadurch gekennzeichnet ist, dass**
die Gewebeeingriffsflächen (1032, 1042, 1052, 1033) der Elektroden (1021, 1031) als eine Reihe Längsstreifen angeordnet sind, die sich von einem proximalen Ende des Backenelements (280, 282, 1010, 1020) zu einem distalen Ende davon erstrecken, wobei der erste, zweite und dritte Streifen der Reihe im Wesentlichen relativ zueinander versetzt sind.

2. Bipolare elektrochirurgische Zange (10) nach Anspruch 1, bei der die Gewebeeingriffsflächen (1032, 1042, 1052, 1033) der Elektroden (1021, 1031) als Längsstreifenpaare angeordnet sind, die sich von einem proximalen Ende eines jeden Backenelements (280, 282, 1010, 1020) zu einem distalen Ende davon erstrecken.

3. Bipolare elektrochirurgische Zange (10) nach Anspruch 2, bei der mindestens ein quer ausgerichteter Kanal zwischen jeweiligen Gewebeeingriffsflächen (1032, 1042, 1052, 1033) auf mindestens einem Backenelement (280, 282, 1010, 1020) definiert ist.

4. Bipolare elektrochirurgische Zange (10) nach einem der vorstehenden Ansprüche, bei der jede Gewebeeingriffsfläche (1032, 1042, 1052, 1033) der Elektrode (1021, 1031) des ersten oder zweiten Backenelements (280, 282, 1010, 1020) einzeln mit einem Generator (350) verbunden ist.

## Revendications

1. Forceps électro-chirurgical bipolaire (10), comprenant :
des premier et second éléments de mâchoire opposés (280, 282, 1010, 1020) ayant des surfaces de mise en prise avec le tissu respectives (1032, 1042, 1052, 1033) associées à ceux-ci, les premier et second éléments de mâchoire (280, 282, 1010, 1020) étant conçus pour un mouvement relatif entre une position ouverte pour recevoir le tissu et une position fermée venant en prise avec le tissu entre lesdites surfaces de mise en prise avec le tissu (1032, 1042, 1052, 1033) afin d'effectuer un scellement du tissu lors de l'activation du forceps (10) ;
les premier et second éléments de mâchoire (280, 282, 1010, 1020) incluant chacun une électrode (1021, 1031) ayant une pluralité de surfaces de mise en prise avec le tissu (1032, 1042, 1052, 1033) qui définissent au moins un canal entre elles,
la pluralité de surfaces de mise en prise avec le tissu (1032, 1042, 1052) du premier élément de mâchoire (280, 1010) étant sensiblement alignées avec la pluralité de surfaces de mise en prise avec le tissu (1033) du deuxième élément de mâchoire (280, 1020) de manière à empêcher un écoulement du fluide entre celles-ci et à forcer le fluide du tissu à s'écouler dans le au moins un canal durant le processus de scellement, où le forceps électro-chirurgical bipolaire (10) est **caractérisé en ce que**
les surfaces de mise en prise avec le tissu (1032, 1042, 1052, 1033) des électrodes (1021, 1031) sont disposées comme un série de bandes longitudinales s'étendant d'une extrémité proximale de chaque élément de mâchoire (280, 282, 1010, 1020) à une extrémité distale de celui-ci, les première, deuxième et troisième bandes de la série étant sensiblement décalées les unes relativement aux autres.

2. Forceps électro-chirurgical bipolaire (10) selon la revendication 1, dans lequel les surfaces venant en prise avec le tissu (1032, 1042, 1052, 1033) des électrodes (1021, 1031) sont disposées comme des paires de bandes longitudinales s'étendant d'une extrémité proximale de chaque élément de mâchoire (280, 282, 1010, 1020) à une extrémité distale de celui-ci.

3. Forceps électro-chirurgical bipolaire (10) selon la revendication 2, dans lequel au moins un canal orienté transversalement est défini entre des surfaces de mise en prise avec le tissu respectives (1032, 1042, 1052, 1033) sur au moins un élément de mâchoire (280, 282, 1010, 1020).

4. Forceps électro-chirurgical bipolaire (10) selon l'une quelconque des revendications précédentes, dans lequel chaque surface de mise en prise avec le tissu (1032, 1042, 1052, 1033) de l'électrode (1021, 1031) des premier et second éléments de mâchoire (280, 282, 1010, 1020) est individuellement connectée à un générateur (350).
